(19) **Europäisches Patentamt / European Patent Office / Office européen des brevets**

(11) **EP 4 610 295 A1**

(12) **EUROPEAN PATENT APPLICATION**
published in accordance with Art. 153(4) EPC

(43) Date of publication:
03.09.2025   Bulletin 2025/36

(21) Application number: 23882736.4

(22) Date of filing: 26.10.2023

(51) International Patent Classification (IPC):
*C08G 69/46* (2006.01)          *C07C 51/06* (2006.01)
*C07C 55/14* (2006.01)          *C07C 209/00* (2006.01)
*C07C 211/12* (2006.01)          *C08G 69/28* (2006.01)
*C08G 69/44* (2006.01)          *C08G 69/48* (2006.01)
*C08J 11/16* (2006.01)

(52) Cooperative Patent Classification (CPC):
C07C 51/06; C07C 55/14; C07C 209/00;
C07C 211/12; C08G 69/28; C08G 69/44;
C08G 69/46; C08G 69/48; C08J 11/16; Y02W 30/62

(86) International application number:
PCT/JP2023/038787

(87) International publication number:
WO 2024/090534 (02.05.2024 Gazette 2024/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority:  27.10.2022  JP 2022172430
27.04.2023  JP 2023073137

(71) Applicant: Toray Industries, Inc.
Tokyo 103-8666 (JP)

(72) Inventors:
• NISHIMURA, Mihoko
Nagoya-shi, Aichi 455-8502 (JP)
• YAMASHITA, Kohei
Nagoya-shi, Aichi 455-8502 (JP)
• YOSHIDOMI, Shinichiro
Nagoya-shi, Aichi 455-8502 (JP)
• KATO, Koya
Nagoya-shi, Aichi 455-8502 (JP)
• UTAZAKI, Kenichi
Nagoya-shi, Aichi 455-8502 (JP)

(74) Representative: Mewburn Ellis LLP
Aurora Building
Counterslip
Bristol BS1 6BX (GB)

(54) **DIAMINE OR DIAMINE COMPOSITION, POLYAMIDE, MOLDED ARTICLE, FIBERS, FILM, SHEET, METHOD FOR PRODUCING DIAMINE AND/OR DICARBOXYLIC ACID, METHOD FOR PRODUCING DIAMINE AND/OR DIAMINE COMPOSITION, AND METHOD FOR PRODUCING POLYAMIDE**

(57)     An object of the present invention is to provide a diamine or a diamine composition having a low content of an amino alcohol compound as a polymerization-inhibiting component in a diamine obtained by depolymerizing a polyamide, and a method for producing a diamine and a dicarboxylic acid, the method being capable of providing the diamine and the dicarboxylic acid with high efficiency and high purity. A diamine or a diamine composition obtained by depolymerizing a polyamide, containing $6.0 \times 10^{-5}$ mol or less of an amino alcohol represented by Chemical formula (1) per 1 g of a diamine:

Chemical formula (1):          $H_2N-R-OH$

wherein R is a residue containing at least one group selected from an aliphatic group, an alicyclic group, and an aromatic group each having 3 to 12 carbon atoms.

EP 4 610 295 A1

## Description

Field

The present invention relates to a diamine or a diamine composition, a polyamide containing the same, and a method for producing a diamine and a dicarboxylic acid. Background

In recent years, interest in global environmental problems has increased with the marine plastic problem as a trigger, and there is a widespread recognition that construction of a sustainable society is necessary. The global environmental problems include global warming, resource depletion, and water shortage. Many of the global environmental problems are caused by an increase in resource consumption and greenhouse gas emission due to the use of fossil fuels and rapid development of industry after the industrial revolution. Therefore, in order to construct a sustainable society, techniques related to the recycling of fossil resources such as plastics and the reduction of greenhouse gas emission are increasingly important.

In particular for polyamide 66 used in a large amount in various fields such as automobiles and electrical and electronic applications, the amount of greenhouse gases emitted at the time of monomer production is large. Therefore, a technique for recycling a product containing polyamide 66 by returning the product to a monomer is desired.

As a technique for recycling a polyamide containing a diamine and a dicarboxylic acid, such as polyamide 66, by depolymerizing and returning the polyamide to a monomer, a method is known in which the polyamide is hydrolyzed in the presence of an inorganic base compound such as sodium hydroxide at a temperature lower than the melting point of polyamide 66, such as 220°C (see, for example, Patent Literature 1 and Non Patent Literature 1).

Further, as a method for carrying out depolymerization of a polyamide containing a diamine and a dicarboxylic acid without using an inorganic base compound, disclosed are a method in which a polyamide is brought into contact with high-temperature and high-pressure water to recover a diamine and a dicarboxylic acid as raw material monomers, and a method in which a polyamide is brought into contact with high-temperature water and then further hydrolyzed using an enzyme to give a diamine and a dicarboxylic acid (see, for example, Patent Literatures 2 and 3 and Non Patent Literature 2).

Citation List

Patent Literatures

Patent Literature 1: JP H07-507556 A
Patent Literature 2: JP 2001-302597 A
Patent Literature 3: WO 2023/149514 A

Non Patent Literatures

Non Patent Literature 1: Chemical Abstract 15 (1959) 14558g
Non Patent Literature 2: Polymer Degradation and Stability 83 (2004) 389-393

Summary

Technical Problem

In the methods disclosed in Patent Literature 2 and Non Patent Literature 2 in which polyamide 66 is decomposed using water in a subcritical state or a supercritical state, the yield of the monomer is at most 25%. Therefore, it is hard to say that the methods are sufficient for achieving recycling use of resources. In addition, Non Patent Literature 2 describes that an oligomer is contained in a reaction mixture, and that a cyclic byproduct is contained in the reaction mixture in an amount equal to or larger than that of the monomer due to a cyclization reaction by deammonification of the diamine in subcritical water and a cyclization reaction by decarboxylation of the dicarboxylic acid in supercritical water. In the method of Patent Literature 3 in which an enzymatic reaction is used, although hexamethylenediamine is obtained in a yield of 82% and adipic acid is obtained in a yield of 72%, it can still not be said that the yield is sufficiently improved through the two-stage hydrolysis step.

According to the method of decomposing polyamide 66 disclosed in Patent Literature 1, polyamide 66 is reacted in an aqueous sodium hydroxide solution at 220°C for 6 hours and then separation and purification are performed to give hexamethylenediamine in a yield of 92% and adipic acid in a yield of 81%. In Non Patent Literature 1, each monomer is obtained in a yield of 90% while the reaction time is shortened to 1 hour as compared with the method of Patent Literature 1, but the method is still not efficient. Furthermore, in Patent Literature 1, although production of a diamine by depolymerization can be confirmed, the literature does not disclose whether or not the diamine has a quality sufficient for use as a raw material of a polyamide.

An object of the present invention is to provide a diamine or a diamine composition having a low content of an amino alcohol compound as a polymerization-inhibiting component in a diamine obtained by depolymerizing a polyamide, and a method for producing a diamine and/or a dicarboxylic acid, the method being capable of providing the diamine and/or the dicarboxylic acid with high efficiency and high purity.

Solution to Problem

To solve the problem, the present invention has the following configuration.

[1] A diamine or a diamine composition obtained by depolymerizing a polyamide, including $6.0 \times 10^{-5}$ mol or less of an amino alcohol represented by Chemical formula (1) per 1 g of a diamine:

Chemical formula (1):          $H_2N$-R-OH

wherein R is a residue containing at least one group selected from an aliphatic group, an alicyclic group, and an aromatic group each having 3 to 12 carbon atoms.

[2] The diamine composition according to [1], wherein an amount of the amino alcohol is $0.010 \times 10^{-5}$ mol or more per 1 g of the diamine.

[3] A polyamide including, as a polymerization raw material, a diamine or a diamine composition obtained by depolymerizing a polyamide, and including $3.0 \times 10^{-5}$ mol/g or less of a terminal structure represented by Chemical formula (2):

Chemical formula (2):          -NH-R-OH

wherein R is a residue containing at least one group selected from an aliphatic group, an alicyclic group, and an aromatic group each having 3 to 12 carbon atoms.

[4] A molded article, a fiber, a film, or a sheet including the polyamide according to [3].

[5] A method for producing a diamine and/or a dicarboxylic acid, the method including:

mixing a polyamide composition (A) containing a polyamide with an aqueous solution (B) of an alkaline (earth) metal compound selected from a hydroxide, an oxide, a carbonate, and a mixture containing two or more thereof to satisfy Formula (1), the polyamide composition (A) containing X mol of a dicarboxylic acid residue, the aqueous solution (B) containing $Y_1$ mol of an alkaline metal ion and/or $Y_2$ mol of an alkaline earth metal ion, and holding a resulting mixture at a temperature of 230°C or higher for 0.1 to 45 minutes:

$$\text{Formula (1): } 1.6 \leq (Y_1 + 2 \times Y_2)/X < 2.4.$$

[6] The production method according to [5], wherein a temperature in the holding is equal to or higher than a melting point of the polyamide composition (A) that is determined by differential scanning calorimetry and lower than 400°C.

[7] The production method according to [5], wherein in a mass ratio of the polyamide composition (A) to the aqueous solution (B) of the alkaline (earth) metal compound selected from the hydroxide, the oxide, the carbonate, and the mixture containing two or more thereof, that is, 1 : Z, Z is 1.0 or more and 10.0 or less.

[8] The production method according to [5], wherein each of the diamine and the dicarboxylic acid is produced from the polyamide composition (A) in a yield of 70 mol% or more.

[9] The production method according to [5], wherein when amounts of a diamine and an amino alcohol represented by Chemical formula (1) that are contained in a reaction mixture (C) obtained by contacting the polyamide composition (A) with the aqueous solution (B) of the alkaline (earth) metal compound selected from the hydroxide, the oxide, the carbonate, and the mixture containing two or more thereof are represented by X' mol and Y' mol, respectively, Y'/X' is $4.0 \times 10^{-3}$ or less:

Chemical formula (1):          $H_2N$-R-OH

wherein R is a residue containing at least one group selected from an aliphatic group, an alicyclic group, and an aromatic group each having 3 to 12 carbon atoms.

[10] The production method according to [5], wherein the polyamide composition (A) is a waste.

[11] A method for producing the diamine or the diamine composition according to [1], the method including the following steps (1) to (3) in this order:

(1) bringing a polyamide composition (A) into contact with an aqueous solution (B) of an alkaline (earth) metal compound selected from a hydroxide, an oxide, a carbonate, and a mixture containing two or more thereof to produce a diamine and a dicarboxylate;

(2) separating the diamine and the dicarboxylate produced in step (1) from each other; and

(3) distilling the diamine separated in step (2).

[12] A method for producing a polyamide, the method including polycondensing a raw material containing the diamine or the diamine composition obtained by the method according to [11].

Advantageous Effects of Invention

According to the present invention, it is possible to provide a diamine or a diamine composition that can be suitably used as a repolymerization raw material and that contains a small amount of an amino alcohol that is a polymerization-inhibiting component. Furthermore, an aqueous solution of an alkaline (earth) metal compound selected from a hydroxide, an oxide, a carbonate, and a mixture containing two or more thereof is used, the aqueous solution containing a specific amount of an alkaline (earth) metal ion with respect to a polyamide composition, and the aqueous solution is reacted with the polyamide composition at 230°C or higher for a short time.

Therefore, it is possible to reduce the production amount of an amino alcohol, and to provide a method for producing a diamine and a dicarboxylic acid in a short time and with high efficiency.

Description of Embodiments

Hereinafter, the present invention will be described in detail with reference to embodiments.

The diamine or the diamine composition of the present invention is a diamine or a diamine composition obtained by depolymerizing a polyamide, and contains $6.0 \times 10^{-5}$ mol or less of an amino alcohol represented by Chemical formula (1) per 1 g of a diamine:

Chemical formula (1):         $H_2N\text{-}R\text{-}OH$

wherein R is a residue containing at least one group selected from an aliphatic group, an alicyclic group, and an aromatic group each having 3 to 12 carbon atoms.

Here, the amino alcohol represented by Chemical formula (1) is a specific compound produced when a polyamide is depolymerized. Specifically, the amino alcohol is a compound produced by conversion of one amino group of the diamine component, which is contained in the polyamide to be depolymerized, into a hydroxyl group. The amino alcohol represented by Chemical formula (1) tends to be produced when a polyamide is depolymerized with high-temperature and high-pressure water.

In the present invention, the polyamide to be depolymerized is a polymer or copolymer having, as a main structural unit, diamine and dicarboxylic acid residues. Here, the phrase "have as a main structural unit" refers to having 50 mol% or more of the diamine and dicarboxylic acid residues in all the structural units, and in a preferable aspect, the polyamide has 80 mol% or more of the residues.

In the present invention, the polyamide to be depolymerized is preferably a homopolymer or copolymer obtained by polycondensation of a diamine and a dicarboxylic acid. Typical examples of the diamine include aliphatic diamines such as tetramethylenediamine, pentamethylenediamine, hexamethylenediamine, 2-methylpentamethylenediamine, nonamethylenediamine, decamethylenediamine, undecamethylenediamine, dodecamethylenediamine, 2,2,4-/2,4,4-trimethylhexamethylenediamine, and 5-methylnonamethylenediamine, aromatic diamines such as metaxylylenediamine and paraxylylenediamine, and alicyclic diamines such as 1,3-bis(aminomethyl)cyclohexane, 1,4-bis(aminomethyl)cyclohexane, 1-amino-3-aminomethyl-3,5,5-trimethylcyclohexane, bis(4-aminocyclohexyl)methane, bis(3-methyl-4-aminocyclohexyl)methane, 2,2-bis(4-aminocyclohexyl) propane, bis(aminopropyl)piperazine, and aminoethyl piperazine. Typical examples of the dicarboxylic acid include aliphatic dicarboxylic acids such as adipic acid, suberic acid, azelaic acid, sebacic acid, undecanedioic acid, and dodecanedioic acid, aromatic dicarboxylic acids such as terephthalic acid, isophthalic acid, 2-chloroterephthalic acid, 2-methylterephthalic acid, 5-methylisophthalic acid, 5-sodium sulfoisophthalic acid, 2,6-naphthalenedicarboxylic acid, hexahydroterephthalic acid, and hexahydroisophthalic acid, and alicyclic dicarboxylic acids such as 1,4-cyclohexanedicarboxylic acid, 1,3-cyclohexanedicarboxylic acid, 1,2-cyclohexanedicarboxylic acid, and 1,3-cyclopentanedicarboxylic acid. In the present invention, two or more of polyamide homopolymers or copolymers derived from these raw materials may be blended.

Examples of the structure of R in the compound (1) obtained by depolymerizing the polyamide include an aliphatic group, an alicyclic group, and an aromatic group each having 3 to 12 carbon atoms.

In the present invention, the polyamide to be depolymerized may be a polyamide copolymer obtained by copolymerizing an amino acid and a lactam, or a blend of a polyamide homopolymer or copolymer obtained by polycondensation of an amino acid and a lactam as long as the object of the present invention is not impaired. Typical examples of the raw material include amino acids such as 6-aminocaproic acid, 11-aminoundecanoic acid, 12-aminododecanoic acid, and para-aminomethylbenzoic acid, and lactams such as ε-caprolactam and ω-laurolactam.

Specific examples of the polyamide include polyhexamethylene adipamide (nylon 66), polytetramethylene adipamide (nylon 46), polytetramethylene sebacamide (nylon 410), polypentamethylene adipamide (nylon 56), polypentamethylene sebacamide (nylon 510), polyhexamethylene sebacamide (nylon 610), polyhexamethylene dodecamide (nylon 612), polydecamethylene adipamide (nylon 106), polydecamethylene sebacamide (nylon 1010), polydecamethylene dodecamide (nylon 1012), polycaproamide/polyhexamethylene adipamide copolymer (nylon 6/66), polycaproamide/polyhexamethylene terephthalamide copolymer (nylon 6/6T), polyhexamethylene adipamide/polyhexamethylene terephthalamide copolymer (nylon 66/6T), polyhexamethylene adipamide/polyhexamethylene isophthalamide copolymer (nylon 66/6I), polyhexamethylene terephthalamide/polyhexamethylene isophthalamide copolymer (nylon 6T/6I), polyhexamethylene terephthalamide/polydodecanamide copolymer (nylon 6T/12), polyhexamethylene adipamide/polyhexamethylene terephthalamide/polyhexamethylene isophthalamide copolymer (nylon 66/6T/6I), polyxylylene adipamide (nylon XD6), polyxylylene sebacamide (nylon XD10), polyhexamethylene terephthalamide/polypentamethylene terephthalamide copolymer (nylon 6T/5T), polyhexamethylene terephthalamide/poly-2-methylpentamethylene terephthalamide copolymer (nylon 6T/M5T), polypentamethylene terephthalamide/polydecamethylene terephthalamide copolymer (nylon 5T/10T), polynonamethylene terephthalamide (nylon 9T), polydecamethylene terephthalamide (nylon 10T), polydodecamethylene terephthalamide (nylon 12T), and copolymers thereof. Two or more of these may be blended. Here, "/" represents that the polyamide is a copolymer, and the same applies hereinafter.

The polyamide to be depolymerized in the present invention may be a polyamide composition containing a polymerization catalyst, a thermoplastic resin other than the polyamide, a dye, various additives, a fibrous filler, a non-fibrous filler, and the like as long as the object of the present invention is not impaired. Here, the fibrous filler is not particularly limited as long as it has a fibrous shape. Examples of the fibrous filler include glass fibers, carbon fibers, potassium titanate whiskers, zinc oxide whiskers, aluminum borate whiskers, aramid fibers, alumina fibers, silicon carbide fibers, ceramic fibers, asbestos fibers, gypsum fibers, and metal fibers. Meanwhile, examples of the non-fibrous filler include wollastonite, zeolite, sericite, kaolin, mica, talc, clay, pyrophyllite, bentonite, montmorillonite, asbestos, aluminosilicate, alumina, silicon oxide, magnesium oxide, zirconium oxide, titanium oxide, iron oxide, calcium carbonate, magnesium carbonate, dolomite, calcium sulfate, barium sulfate, magnesium hydroxide, calcium hydroxide, aluminum hydroxide, glass beads, ceramic beads, boron nitride, silicon carbide, and silica. Two or more of these may be blended.

The polyamide to be depolymerized in the present invention may be a resin molded article containing a polyamide. Examples of the resin molded article containing a polyamide include a polyamide product, an industrial waste generated in the process of producing a polyamide product, and a waste that is a used polyamide product. Specific examples of the polyamide product include automobile components such as an engine peripheral component such as a radiator tank and an oil pan and a gear, electrical and electronic components such as a connector and a switch, components for industrial machine applications such as a fastener and a binding band, industrial fiber structures such as an airbag, clothing fiber structures, sheets, films, and molded articles. Further, the polyamide may also be product debris, pellet debris, massive debris, or the like generated in the production process of these products.

In the method for producing a diamine and/or a dicarboxylic acid of the present invention, the polyamide composition (A) is preferably a waste. When the polyamide composition (A) is a waste, fossil resources can be recycled. Furthermore, since there is no need to incinerate a waste, greenhouse gas emission can be reduced.

The diamine or the diamine composition of the present invention contains $6.0 \times 10^{-5}$ mol or less of an amino alcohol represented by Chemical formula (1) per 1 g of a diamine:

$$\text{Chemical formula (1):} \qquad \text{H}_2\text{N-R-OH}$$

wherein R is a residue containing at least one group selected from an aliphatic group, an alicyclic group, and an aromatic group each having 3 to 12 carbon atoms.

When the diamine or diamine composition obtained in the present invention is reused as a polyamide raw material, the amino alcohol represented by Chemical formula (1) contained in a specific amount in the diamine or diamine composition serves as a terminal capping agent and inhibits polymerization. An object of the present invention is to regenerate the diamine or diamine composition to a material of a practically usable quality that allows repolymerization. When the amount of the amino alcohol represented by Chemical formula (1) contained in the diamine or diamine composition is $6.0 \times 10^{-5}$ mol or less per 1 g of a diamine, the polymerization degree of the polyamide can be increased to a practically usable level. The amount of the amino alcohol is preferably $2.0 \times 10^{-5}$ mol or less, more preferably $1.0 \times 10^{-5}$ mol or less, and still more preferably $0.5 \times 10^{-5}$ mol or less per 1 g of a diamine. Meanwhile, the present inventors have found that when a regenerated diamine, which contains at least $0.010 \times 10^{-5}$ mol (detection limit of gas chromatography described in Examples) of an amino alcohol in the diamine composition per 1 g of a diamine, is polymerized, the resulting polyamide has a polymerization degree higher than that of a polyamide obtained by polymerizing a petroleum-derived diamine under the same conditions. It is considered that this is because a trace amount of the amino alcohol has a small influence as the terminal capping agent described above and acts like a plasticizer to decrease the melt viscosity of the polyamide, thereby increasing the molecular mobility and promoting the polymerization reaction. In the present invention, the amount of the amino alcohol represented by Chemical formula (1) contained in the diamine or diamine composition is measured according to quantitative analysis by gas chromatography (GC) described in Examples.

The method for producing a diamine or a diamine composition of the present invention preferably includes the steps (1) to (3) in this order:

(1) bringing a polyamide composition (A) into contact with an aqueous solution (B) of an alkaline (earth) metal compound selected from a hydroxide, an oxide, a carbonate, and a mixture containing two or more thereof to produce a diamine and a dicarboxylate;
(2) separating the diamine and the dicarboxylate produced in step (1) from each other; and
(3) distilling the diamine separated in step (2).In the present description, the notation "alkaline (earth) metal compound" means both a compound containing an alkaline metal atom such as lithium, sodium, or potassium, and an alkaline earth atom such as magnesium, calcium, or barium, and mixtures thereof.

The method for producing a diamine and a dicarboxylate in step (1) is not particularly limited. For example, when the depolymerization reaction is performed by a batch method, a reaction mixture containing a diamine and an alkaline (earth) metal dicarboxylate can be obtained after the depolymerization reaction. When the depolymerization reaction is performed by a continuous method, a reaction mixture containing a diamine and an alkaline (earth) metal dicarboxylate can be obtained as the reaction proceeds. The state of the reaction mixture is not particularly limited, and examples thereof include an aqueous solution, a slurry, and a paste.

As a method for separating the diamine and the dicarboxylate from each other in step (2), a known method such as extraction or solid-liquid separation can be selected depending on the properties of the produced diamine and dicarboxylate. When polyamide 66 is used as a polyamide, and an aqueous sodium hydroxide solution is used as an aqueous solution of an alkaline (earth) metal compound selected from a hydroxide, an oxide, a carbonate, and a mixture containing two or more thereof, it is particularly preferable to separate the diamine and the dicarboxylate from each other by extraction because both the diamine and the dicarboxylate are water-soluble. When there is a component insoluble in water in the reaction mixture, it is also possible to separate the component in advance by a known method such as solid-liquid separation and subject the reaction mixture to step (2). When polyamide 610 is used as a polyamide, and sodium hydroxide is used as an aqueous solution of an alkaline (earth) metal compound selected from a hydroxide, an oxide, a carbonate, and a mixture containing two or more thereof, the dicarboxylate can be precipitated by cooling. Therefore, the diamine and the dicarboxylate can be separated from each other by solid-liquid separation.

In step (3), it is preferable to distill the diamine separated from the dicarboxylate in step (2) to reduce the content of the amino alcohol represented by Chemical formula (1). For example, as for a case where polyamide 66 is used as the polyamide, hexamethylenediamine as a raw material monomer of polyamide 66 has a boiling point of 204°C, and 6-amino-1-hexanol that is an amino alcohol produced by conversion of one amino group of hexamethylenediamine into a hydroxyl group has a boiling point of 225°C. The distillation and purification are preferable because the content of the amino alcohol as a polymerization-inhibiting component can be reduced, and the quality of the diamine or diamine composition can be improved to a practically usable level at which repolymerization is possible. The diamine or diamine composition thus obtained can be used as a polymerization raw material of a polyamide, similarly to a diamine and a carboxylic acid produced from petroleum-derived raw materials.

The polyamide of the present invention contains, as a polymerization raw material, a diamine or a diamine composition obtained by depolymerizing a polyamide, and contains $3.0 \times 10^{-5}$ mol/g or less of a terminal structure represented by Chemical formula (2):

Chemical formula (2):            -NH-R-OH

wherein R is a residue containing at least one group selected from an aliphatic group, an alicyclic group, and an aromatic group each having 3 to 12 carbon atoms.

In the present invention, the content of the terminal structure represented by Chemical formula (2) can be quantified by hydrolyzing a polyamide with hydrochloric acid, and then analyzing an amino alcohol trimethylsilylated by N,O-bis(trimethylsilyl)trifluoroacetamide (a BSTFA reagent) by gas chromatography (GC). The content of the terminal structure referred to herein is determined as the molar equivalent of the terminal structure represented by Chemical formula (2) per 1 g of the polyamide. When the content of the terminal structure is in this range, a polyamide having a high polymerization degree at a practically usable level can be obtained. The content is more preferably $1.0 \times 10^{-5}$ mol/g or less, still more preferably $0.5 \times 10^{-5}$ mol/g or less, and most preferably 0 mol/g.

In the present invention, in the polyamide obtained by polymerizing a diamine composition containing about $0.010 \times 10^{-5}$ mol of the amino alcohol represented by Chemical Formula (1) per 1 g of the diamine, the content of the terminal structure represented by Chemical formula (2) was 0 mol/g (below the detection limit of gas chromatography) and an effect was recognized that the polyamide has a polymerization degree higher than that of the polyamide of Reference Example 1 obtained by polymerizing a reagent diamine.

The method for producing a polyamide of the present invention includes the step of polycondensing a raw material containing the diamine or the diamine composition of the present invention.

The polyamide of the present invention can be produced by polycondensation of a diamine or a diamine composition, which is obtained by depolymerizing a polyamide, with a dicarboxylic acid as it is or as a salt thereof using the diamine or diamine composition as one component of raw materials. In the case where a polyamide is produced, the polyamide has a high polymerization degree when the equimolarity between the amount of carboxyl terminal groups and the amount of amino terminal groups of the monomers is appropriately maintained. For the purpose of uniformly mixing the diamine and the dicarboxylic acid, it is preferable that the diamine, the dicarboxylic acid, and a salt thereof are uniformly dissolved in the polymerization system. It is also preferable that the polycondensation reaction is performed with heating. An example of a preferable lower limit of the temperature is 150°C, and an example of a preferable upper limit of the temperature is 400°C.

The polyamide thus obtained can be processed into various products such as an injection molded article, an extrusion molded article, a fiber structure, and a film and used, similarly to a polyamide produced from a petroleum-derived raw material. These products are useful for automobile components, electrical and electronic components, industrial machine applications, industrial fiber structures, clothing fiber structures, sheets, films, and other applications.

The molded article, fiber, film, or sheet of the present invention contains the polyamide of the present invention. The polyamide of the present invention contains, as a polymerization raw material, a diamine or a diamine composition obtained by depolymerizing a polyamide. Therefore, the resulting polyamide can serve as a recycled material that has low environmental burden and contributes to recycling of resources and reduction of greenhouse gas emission.

The method for producing a diamine and a dicarboxylic acid of the present invention includes the step of mixing a polyamide composition (A) containing a polyamide that contains X mol of a dicarboxylic acid residue with an aqueous solution (B) of an alkaline (earth) metal compound selected from a hydroxide, an oxide, a carbonate, and a mixture containing two or more thereof, the aqueous solution (B) containing $Y_1$ mol of an alkaline metal ion and/or $Y_2$ mol of an alkaline earth metal ion, so as to satisfy Formula (1), and holding the resulting mixture at a temperature of 230°C or higher for 0.1 to 45 minutes:

$$\text{Formula (1): } 1.6 \leq (Y_1 + 2 \times Y_2)/X < 2.4.$$

In the present invention, as for the above-mentioned step, the temperature and the time when mixing the polyamide composition (A) with the aqueous solution (B) and holding the resulting mixture at the temperature and the time in the above-mentioned ranges may be referred to as a reaction temperature and a reaction time, respectively. In addition, the pressure at the time of holding the mixture may be referred to as a reaction pressure.

As described above, the method for producing a diamine and/or a dicarboxylic acid of the present invention is characterized by mixing a polyamide composition (A) containing a polyamide with an aqueous solution (B) of an alkaline (earth) metal compound selected from a hydroxide, an oxide, a carbonate, and a mixture containing two or more thereof to produce a diamine and a dicarboxylic acid, which are monomers for a polyamide that constitutes the polyamide composition (A).

Examples of the polyamide composition (A) containing a polyamide include the polyamide to be depolymerized described above, and a composition containing a blend of a polymerization catalyst, a thermoplastic resin other than the polyamide, a dye, various additives, a fibrous filler, a non-fibrous filler, and the like.

The aqueous solution (B) of an alkaline (earth) metal compound selected from a hydroxide, an oxide, a carbonate, and a mixture containing two or more thereof is obtained by blending an alkaline (earth) metal compound with water. Examples of the alkaline (earth) metal hydroxide include lithium hydroxide, sodium hydroxide, potassium hydroxide, magnesium hydroxide, calcium hydroxide, and barium hydroxide. Examples of the alkaline (earth) metal oxide include lithium oxide, sodium oxide, potassium oxide, magnesium oxide, calcium oxide, and barium oxide. The alkaline (earth) metal carbonate may be either a normal salt or an acid salt (hydrogen carbonate). Examples of the normal salt include dilithium carbonate, disodium carbonate, dipotassium carbonate, magnesium carbonate, calcium carbonate, and barium carbonate. Examples of the alkaline metal hydrogen carbonate include lithium hydrogen carbonate, sodium hydrogen carbonate, potassium hydrogen carbonate, magnesium carbonate, calcium carbonate, and barium carbonate. In the present invention, two or more compounds selected from these alkaline (earth) metal hydroxides, alkaline (earth) metal oxides, and alkaline (earth) metal carbonates may be blended and used. As the alkaline (earth) metal, sodium, potassium, and calcium are preferable from the viewpoint of protecting mineral resources, sodium and potassium are more preferable from the viewpoint of increasing the production rate of the diamine and the dicarboxylic acid, and sodium is particularly preferable. In addition, for the purpose of improving the hydrolysis reactivity with the polyamide, normal salts of hydroxides, oxides, and carbonates are preferable, and hydroxides and oxides are particularly preferable. The water used here is not particularly limited, and any water such as tap water, deionized water, distilled water, or well water may be used. From the viewpoint of inhibiting side reactions due to the influence of the coexisting salt, deionized water or distilled water is preferably used as water.

The amount of the alkaline metal ion in the aqueous solution (B) of an alkaline (earth) metal compound selected from a hydroxide, an oxide, a carbonate, and a mixture containing two or more thereof is determined according to the polyamide content in the polyamide composition (A). When the amount of the dicarboxylic acid residue in the polyamide contained in the polyamide composition (A) is defined as X mol, and the amounts of the alkaline metal ion and the alkaline earth metal ion contained in the aqueous solution (B) of an alkaline (earth) metal compound are defined as $Y_1$ mol and $Y_2$ mol, respectively, $(Y_1 + 2 \times Y_2)/X$ is 1.6 or more and less than 2.4. When $(Y_1 + 2 \times Y_2)/$ is controlled in this range, in the hydrolysis of the polyamide composition (A), yields of the diamine and the dicarboxylic acid can be improved, and the amount of the amino alcohol represented by Chemical formula (1) can be reduced. $(Y_1 + 2 \times Y_2)/X$ is preferably 1.8 or more, more preferably 1.9 or more, and particularly preferably 2.0 or more. Meanwhile, $(Y_1 + 2 \times Y_2)/X$ is more preferably 2.2 or less.

The amount of the dicarboxylic acid residue contained in the polyamide composition (A) can be quantified from a spectrum obtained by proton nuclear magnetic resonance spectroscopy ($^1$H-NMR) using sulfuric acid or hexafluoroisopropanol as a deuterated solvent.

In the present invention, both the yields of the diamine and the dicarboxylic acid obtained by depolymerizing the polyamide contained in the polyamide composition (A) are preferably 70 mol% or more. The yields referred to herein are the amounts of the diamine and the dicarboxylic acid contained in the reaction mixture in terms of mole percentage, respectively relative to the numbers of moles of the diamine residue and the dicarboxylic acid residue contained in the polyamide composition (A) that are each defined as 100. Both the yields are preferably 80 mol% or more, more preferably 90 mol% or more, and still more preferably 95 mol% or more. In the present invention, the yield of the diamine is calculated according to quantitative analysis by gas chromatography (GC) described in Examples. In the present invention, the yield of the dicarboxylic acid is calculated according to quantitative analysis by ion chromatography (IC) described in Examples. When the yields of the diamine and the dicarboxylic acid are in the preferable range, the recycling rate can be increased.

The amount of the aqueous solution (B) of an alkaline (earth) metal compound selected from a hydroxide, an oxide, a carbonate, and a mixture containing two or more thereof is not particularly limited. However, in a mass ratio of the polyamide composition (A) to the aqueous solution (B) of an alkaline (earth) metal compound selected from a hydroxide, an oxide, a carbonate, and a mixture containing two or more thereof, that is, 1 : Z, Z is preferably 1 or more. If Z is less than 1, when the polyamide composition (A) is mixed with the aqueous solution (B) of an alkaline (earth) metal compound selected from a hydroxide, an oxide, a carbonate, and a mixture containing two or more thereof at 230°C or higher, the dispersibility and solubility of the polyamide composition (A) in water decrease, and the reaction efficiency tends to decrease. Z is more preferably 2.0 or more, and still more preferably 3.0 or more. Meanwhile, Z is preferably 10 or less, more preferably 8.0 or less, and still more preferably 6.0 or less. The present invention relates to a highly efficient method for producing a diamine and a dicarboxylic acid by depolymerizing the polyamide composition (A) for the purpose of realizing both recycling of fossil resources and reduction of greenhouse gas emission. Water has a specific heat capacity of 4.3 kJ/kg·K and a heat of vaporization of 2,250 kJ/kg, which are very high as compared to those of other organic solvents, and therefore, it is important to reduce the amount of water used. When the amount of the aqueous solution (B), which contains an alkaline (earth) metal compound selected from a hydroxide, an oxide, a carbonate, and a mixture containing two or more thereof in an amount in a certain range, is in the above-mentioned range, both the production efficiency of the diamine and the dicarboxylic acid and energy saving can be realized.

The method for preparing the aqueous solution (B) of an alkaline (earth) metal compound selected from a hydroxide, an oxide, a carbonate, and a mixture containing two or more thereof is not particularly limited, and the aqueous solution may be prepared by mixing the alkaline (earth) metal compound and water in advance, or the aqueous solution may be prepared by mixing the alkaline (earth) metal compound and water when being brought into contact with the polyamide composition (A) in a reaction vessel.

The method for producing a diamine and/or a dicarboxylic acid of the present invention is characterized by bringing the polyamide composition (A) into contact with the aqueous solution (B) of an alkaline (earth) metal compound selected from a hydroxide, an oxide, a carbonate, and a mixture containing two or more thereof at a temperature of 230°C or higher for 0.1 to 45 minutes to produce a diamine and a dicarboxylic acid.

In the method for producing a diamine and/or a dicarboxylic acid of the present invention, the reaction time of the reaction between the polyamide composition (A) and the aqueous solution (B) of an alkaline (earth) metal compound selected from a hydroxide, an oxide, a carbonate, and a mixture containing two or more thereof is 0.1 to 45 minutes. As described above, the reaction time in the present invention refers to the total time for holding the mixture at a temperature of 230°C or higher. As for a heating process until the temperature reaches the reaction temperature and a cooling process after the reaction at the reaction temperature, the time during which the polyamide composition (A) and the aqueous solution (B) of an alkaline (earth) metal compound selected from a hydroxide, an oxide, a carbonate, and a mixture containing two or more thereof are mixed at a temperature of 230°C or higher in a reaction vessel is included in the reaction time. A reaction time exceeding 45 minutes is not preferable because the yields of the diamine and the dicarboxylic acid decrease and the production amount of the amino alcohol increases. The reaction time is preferably 45 minutes or less, more preferably 40 minutes or less, and still more preferably 35 minutes or less. Meanwhile, if the reaction time is less than 0.1 minutes, it is difficult to make the depolymerization reaction sufficiently proceed, and the yields of the diamine and the dicarboxylic acid decrease. The reaction time is preferably 0.1 minutes or more, more preferably 1 minute or more, and still more preferably 3 minutes or more.

Bringing the polyamide composition (A) into contact with the aqueous solution (B) of an alkaline (earth) metal compound selected from a hydroxide, an oxide, a carbonate, and a mixture containing two or more thereof for a reaction time in the above-mentioned preferable range can reduce the amount of the amino alcohol represented by Chemical formula (1). It is considered that the amino alcohol is produced by a nucleophilic substitution reaction in which one $NH_2$ group of the diamine that is a depolymerization product of the polyamide composition (A) is converted into an OH group. As for the amount of the amino alcohol represented by Chemical formula (1) in the present invention, when the amounts of the diamine and the amino alcohol represented by Chemical formula (1) that are contained in a reaction mixture (C) obtained after the depolymerization are represented by X' mol and Y' mol, respectively, Y'/X' is preferably $4.0 \times 10^{-3}$ or less. Y'/X' is more preferably $3.0 \times 10^{-3}$ or less, still more preferably $2.0 \times 10^{-3}$ or less, and particularly preferably $1.0 \times 10^{-3}$ or less. The amounts of the diamine and the amino alcohol represented by Chemical formula (1) can be calculated according to quantitative analysis by gas chromatography (GC) described in Examples. When the amount of the amino alcohol represented by Chemical formula (1) is in a preferable range, a polyamide that is obtained by repolymerizing a diamine or a diamine composition, which is obtained by subsequent purification, can have a high molecular weight.

The reaction temperature in the method for producing a diamine and/or a dicarboxylic acid of the present invention is 230°C or higher. The reaction temperature is the temperature during the contact between the polyamide composition (A) and the aqueous solution (B) of an alkaline (earth) metal compound selected from a hydroxide, an oxide, a carbonate, and a mixture containing two or more thereof in a temperature-controlled reaction vessel, and may be a constant temperature or a temperature varied over time. Incidentally, the temperature in the heating process until the temperature reaches the reaction temperature and the cooling process after the reaction at the reaction temperature is not particularly limited as long as it is lower than 230°C. Since the reaction temperature is 230°C or higher, the time required for the hydrolysis reaction of the polyamide composition (A) can be shortened, and the production efficiency of the diamine and the dicarboxylic acid can be improved.

In addition, the reaction temperature is preferably a temperature equal to or higher than the melting point of the polyamide composition (A) that is determined by differential scanning calorimetry and lower than 400°C. When the reaction temperature is equal to or higher than the melting point of the polyamide composition (A), the polyamide composition (A) is easily dispersed and dissolved in water, and the reaction efficiency can be improved. Water is in a state of neither liquid nor gas when the pressure is increased to 22.1 MPa and the temperature is increased to 374.2°C. Water in this state is referred to as supercritical water, and has both a dissolving capability as a liquid and a diffusion capability as a gas. Therefore, the supercritical water is attracting attention as a reaction field for decomposing a polymer. Hot water under a temperature and a pressure slightly lower than the critical point temperature and pressure of water and in the vicinity of the critical point is referred to as subcritical water. Although the subcritical water is water, it has characteristics that (i) it has low dielectric constant and (ii) it has high ionic product. The dielectric constant and the ionic product of the subcritical water depend on the temperature and the partial pressure of water, and can be controlled. Since the subcritical water has low dielectric constant, it serves as a good solvent for organic compounds even though it is water. Moreover, since the subcritical water has high ionic product, hydrogen ions and hydroxide ions are generated at high concentrations, so that the subcritical water has an excellent hydrolysis action. When the reaction temperature is lower than 400°C, it is possible to inhibit an undesirable reaction such as thermal decomposition while utilizing a reaction field having excellent characteristics of supercritical water or subcritical water at the time of mixing and reacting the polyamide resin (A) with the aqueous solution (B) of an alkaline (earth) metal compound selected from a hydroxide, an oxide, a carbonate, and a mixture containing two or more thereof. Meanwhile, a reaction temperature lower than 374.2°C is preferable because the ionic product can be preferably controlled using the subcritical state of water, so that the reaction efficiency can be improved and the reaction time can be shortened. Furthermore, for the purpose of inhibiting an overreaction of the diamine and the dicarboxylic acid, the reaction temperature is more preferably lower than 350°C, and still more preferably lower than 300°C. Meanwhile, for the purpose of accelerating the depolymerization reaction, the reaction temperature is more preferably 250°C or higher, and still more preferably 270°C or higher.

The melting point of the polyamide composition (A) in the present invention referred to herein is the temperature of an endothermic peak that appears when the temperature is raised from 40°C to 300°C at a heating rate of 10°C/min under a nitrogen flow using a differential scanning calorimeter manufactured by TA Instruments. When two or more endothermic peaks are detected, the temperature of the endothermic peak detected on the highest temperature side is defined as the melting point. A melting point of the polyamide composition (A) exceeding 400°C is not preferable because a thermal decomposition reaction or the like of the polyamide composition (A) proceeds, so that the recovery rates of the diamine and the dicarboxylic acid are reduced. The lower limit of the melting point of the polyamide composition (A) is not particularly limited, but a preferable lower limit can be, for example, 50°C. When the melting point of the polyamide composition (A) is 50°C or higher, the polyamide is solid at normal temperature and is excellent in handleability.

The reaction pressure is, for example, preferably higher than the saturated vapor pressure. When the reaction pressure is set higher than the saturated vapor pressure, water can maintain a liquid state and the reaction easily proceeds. Therefore, the pressure of the aqueous solution (B) of an alkaline (earth) metal compound selected from a hydroxide, an oxide, a carbonate, and a mixture containing two or more thereof is preferably higher than the saturated vapor pressure. The upper limit of the reaction pressure is not particularly limited, but the reaction pressure may be, for example, 22.1 MPa or less. A reaction pressure in such a range is preferable because the ionic product of water described above tends to increase. Examples of the method for setting the reaction pressure in such a pressure range include a method of pressurizing and sealing the inside of a pressure vessel. In order to pressurize the inside of the pressure vessel, a gas should be enclosed in addition to the polyamide composition (A) and the aqueous solution (B) of an alkaline (earth) metal compound selected from a hydroxide, an oxide, a carbonate, and a mixture containing two or more thereof. Examples of the gas to be enclosed include air, argon, and nitrogen. From the viewpoint of inhibiting side reactions such as an oxidation reaction, it is preferable to use nitrogen or argon as the gas to be enclosed. The inside of the pressure vessel can also be pressurized by introducing high-pressure water. When high-pressure water is used, a state in which there is no gas in the pressure vessel can be adopted. The degree of gas pressurization is not particularly limited since the pressure is set to a target pressure, but the pressure may be 0.3 MPa or more.

In the present invention, for mixing the polyamide composition (A) with the aqueous solution (B) of an alkaline (earth) metal compound selected from a hydroxide, an oxide, a carbonate, and a mixture containing two or more thereof, various known reaction methods such as a batch method and a continuous method can be adopted. Examples of the batch method include an autoclave and a vertical or horizontal reactor each having a stirrer and a heating function, and a vertical or horizontal reactor having a compression mechanism such as a cylinder in addition to a stirrer and a heating function. Examples of the continuous method include an extruder, a tubular reactor, a tubular reactor equipped with a mixing mechanism such as a baffle, a line mixer, a vertical or horizontal reactor, a vertical or horizontal reactor equipped with a stirrer, and a tower, each having a heating function. The atmosphere in the production is preferably a non-oxidizing atmosphere, more preferably an inert atmosphere such as nitrogen, helium, or argon, and still more preferably a nitrogen atmosphere from the viewpoint of economy and ease of handling.

The method for recovering the diamine and the dicarboxylic acid produced by the method of the present invention is not particularly limited, and any method can be employed. For example, when the depolymerization reaction is performed by a batch method, a reaction mixture (C) of the diamine and the alkaline (earth) metal dicarboxylate can be obtained after the depolymerization reaction. When the depolymerization reaction is performed by a continuous method, a reaction mixture (C) of the diamine and the alkaline (earth) metal dicarboxylate can be obtained as the reaction proceeds. The diamine and the alkaline (earth) metal dicarboxylate in the obtained reaction mixture (C) are separated from each other by a known method such as extraction, the separated alkaline (earth) metal dicarboxylate is converted into a dicarboxylic acid under acidic conditions, and the diamine and the dicarboxylic acid can be individually recovered by a known method such as distillation/solid-liquid separation. In addition, when the reaction mixture (C) contains a component insoluble in water, it is possible to separate the component in advance by a known method such as solid-liquid separation and subject the reaction mixture (C) to the step of recovering the diamine and the dicarboxylic acid. For the purpose of reducing the content of the amino alcohol in the diamine, it is particularly preferable to distill and purify the diamine.

In order to obtain the diamine and the dicarboxylic acid of higher purity, purification may be further performed by a known method.

The diamine and the dicarboxylic acid obtained by the method for producing a diamine and/or a dicarboxylic acid of the present invention can be used as a polymerization raw material of a polyamide, similarly to a diamine and a carboxylic acid produced from petroleum-derived raw materials. A polyamide resin can be produced by heat polycondensation of the diamine and/or the dicarboxylic acid as it is or as a salt thereof. In the present invention, a polyamide can be regenerated by repolymerizing a diamine and/or a dicarboxylic acid obtained by depolymerizing a polyamide. Therefore, the polyamide can serve as a recycled material that has low environmental burden and contributes to recycling of resources and reduction of greenhouse gas emission.

In addition, the polyamide thus obtained can be processed into various products such as an injection molded article, an extrusion molded article, a fiber structure, and a film and used, similarly to a polyamide produced from a petroleum-derived raw material. These products are useful as automobile components, electrical and electronic components, uses for industrial machine applications, industrial fiber structures, clothing fiber structures, sheets, films, and other applications.

Examples

Hereinafter, the present invention will be described with reference to examples, but the present invention is not limited by these examples.

In the examples, the following raw materials were used.

(A-1) Polyamide 66: "AMILAN" (registered trademark) CM3001-N manufactured by Toray Industries, Inc., melting point: 262°C

(A-2) Polyamide 66 waste: airbag base fabric made of polyamide 66 recovered from an end-of-life automobile

(A-3) Polyamide 66 waste: silicone-coated airbag base fabric made of polyamide 66 recovered from an end-of-life automobile, estimated silicone adhesion amount: 25 g/m$^2$

(A-4) Polyamide 66 waste: GF30% reinforced polyamide 66 molding debris (crushed sprue and runner)

Sodium hydroxide: special grade reagent, manufactured by KANTO CHEMICAL CO., INC.

Sodium carbonate: special grade reagent, manufactured by KANTO CHEMICAL CO., INC.

Sodium hydrogen carbonate: first grade, manufactured by FUJIFILM Wako Pure Chemical Corporation

Adipic acid: special grade, manufactured by FUJIFILM Wako Pure Chemical Corporation

Hexamethylenediamine: first grade, manufactured by FUJIFILM Wako Pure Chemical Corporation

<<Evaluation Method>>

(Amount of amino alcohol in diamine composition (GC))

The amount of the amino alcohol in the diamine composition was calculated according to quantitative analysis by gas chromatography (GC).

Apparatus: GC-2010 manufactured by Shimadzu Corporation

Column: DB-5, 0.32 mm × 30 m (0.25 μm) manufactured by Agilent Technologies

Carrier gas: helium

Detector: flame ionization detector (FID)

Sample: A sample for gas chromatography measurement was prepared by taking about 0.02 g of the diamine composition, diluting the diamine composition with about 10 g of deionized water, and separating and removing components insoluble in deionized water by filtration.

Quantification of amino alcohol: The amount of the amino alcohol was quantified by an absolute calibration curve method.

(Diamine yield, amount of amino alcohol in reaction mixture (GC))

The amount of the diamine and the amount of the amino alcohol in the reaction mixture (C) were calculated according to quantitative analysis by gas chromatography (GC), and the diamine yield was determined from the calculated amount of diamine.

Apparatus: GC-2010 manufactured by Shimadzu Corporation

Column: DB-5, 0.32 mm × 30 m (0.25 μm) manufactured by Agilent Technologies

Carrier gas: helium

Detector: flame ionization detector (FID)

Sample: A sample for gas chromatography measurement was prepared by taking about 0.15 g of the reaction mixture, diluting the reaction mixture with about 10 g of deionized water, and separating and removing components insoluble in deionized water by filtration.

Quantification of diamine: The amount of the diamine was quantified by an absolute calibration curve method.

Quantification of amino alcohol: The amount of the amino alcohol was quantified by an absolute calibration curve method.

(Yield of dicarboxylic acid (IC))

The dicarboxylic acid yield was calculated according to quantitative analysis of sodium dicarboxylate in the reaction mixture (C) by ion chromatography (IC).

> Apparatus: HIC-20Asuper manufactured by Shimadzu Corporation
> Column: Shim-pack IC-SA2 (250 mm × 4.6 mm ID) manufactured by Shimadzu Corporation
> Detector: electrical conductivity detector (suppressor)
> Eluent: aqueous solution of 4.0 mM sodium hydrogen carbonate/1.0 mM sodium carbonate
> Flow rate: 1.0 ml/min
> Injection volume: 50 microliters
> Column temperature: 30°C
> Sample: A sample for ion chromatography measurement was prepared by taking about 0.02 g of the reaction mixture, diluting the reaction mixture with about 10 g of deionized water, and separating and removing components insoluble in deionized water by filtration.
> Quantification of dicarboxylic acid: The amount of sodium dicarboxylate was quantified by an absolute calibration curve method.

(Melting point of polyamide (DSC))

About 5.0 mg of a polyamide was heated from 40°C to 300°C at a heating rate of 10°C/min under a nitrogen flow using a differential thermal analyzer (TG/DTA7200 manufactured by Hitachi High-Tech Science Corporation). The temperature of an endothermic peak appeared then was defined as the melting point. When two or more endothermic peaks were detected, the temperature of the endothermic peak detected on the highest temperature side was defined as the melting point.

(Molecular weight of polyamide)

About 2.5 mg of the polyamide obtained in each of examples and comparative examples was dissolved in 4 ml of hexafluoroisopropanol (to which 0.005 N-sodium trifluoroacetate was added), and the resulting solution was filtered through a 0.45 μm filter. Using the obtained solution, the number average molecular weight (Mn) was measured by GPC measurement. The measurement conditions are shown below.

> Pump: e-Alliance GPC system (manufactured by Waters Corporation)
> Detector: differential refractometer Waters 2414 (manufactured by Waters Corporation)
> Column: Shodex HFIP-806M (two pieces) + HFIP-LG
> Solvent: hexafluoroisopropanol (to which 0.005 N-sodium trifluoroacetate was added)
> Flow rate: 1 ml/min
> Sample injection amount: 0.1 ml
> Temperature: 30°C
> Molecular weight reference substance: polymethyl methacrylate

(Amount of amino alcohol at terminal of polyamide (GC))

About 50 mg of a polyamide resin obtained in each of examples and comparative examples was precisely weighed and put in a glass ampoule having an internal volume of 16 ml, 8 ml of a 6 N aqueous hydrochloric acid solution was added thereto, and then the ampoule was sealed. The ampoule was placed in a pressure-resistant container, heated at 180°C for 20 hours, and subjected to a hydrolysis treatment. After cooling, the ampoule was removed and the contents were concentrated to dryness. Then, hydrochloric acid was removed and the product was dried, and the resulting dried solid was trimethylsilylated using N,O-bis(trimethylsilyl)trifluoroacetamide (a BSTFA reagent). The reaction product was diluted with dehydrated acetonitrile to prepare a sample for gas chromatography measurement. The measurement conditions are shown below.

> Apparatus: GC-2010 manufactured by Shimadzu Corporation
> Column: DB-5, 0.32 mm × 30 m (0.25 μm) manufactured by Agilent Technologies
> Carrier gas: helium
> Detector: flame ionization detector (FID)
> Quantification of amino alcohol: The amount of the amino alcohol was quantified by an absolute calibration curve method using a trimethylsilylated reagent amino alcohol.

[Example 1]

Into an SUS316L autoclave equipped with a stirrer, polyamide 66 (A-1) having a melting point of 262°C and an aqueous sodium hydroxide solution in the amounts shown in Table 1 were charged. The molar ratio $Y_1/X$ represented by the compounding amount X mol of polyamide 66 and the compounding amount $Y_1$ mol of sodium hydroxide was 2.11.
The reaction vessel was purged with nitrogen and sealed under a nitrogen pressure of 0.5 MPa, and then the reaction vessel was held at 280°C for 15 minutes while being stirred at 200 rpm to perform a reaction. During the reaction, the pressure in the system was 6.5 MPa. After completion of the reaction, the reaction mixture was cooled to room temperature and recovered. The total time for mixing the polyamide composition and the aqueous sodium hydroxide solution in the reaction vessel at 230°C or higher was 40 minutes.
As for the recovered reaction mixture, the diamine yield (X') calculated by gas chromatography measurement was 98 mol%, and the dicarboxylic acid yield calculated by high performance liquid chromatography measurement was 95 mol%. The amino alcohol amount (Y') was 0.14 mol%, and Y'/X' was $1.4 \times 10^{-3}$.

[Examples 2 to 9 and Comparative Examples 1 to 3]

Depolymerization of a polyamide was performed in the same manner as in Example 1 except that the kind of the polyamide used as a raw material, the amount and concentration of an aqueous sodium hydroxide solution, the amount of deionized water, the reaction temperature, and the reaction time were appropriately changed. In Comparative Example 3, since the reaction was performed at a temperature lower than 230°C, the reaction time was defined as the time for the treatment at 220°C (60 minutes).

[Examples 10 and 11]

Depolymerization of a polyamide was performed in the same manner as in Example 1 except that sodium hydroxide was changed to sodium carbonate or sodium hydrogen carbonate.
The reaction conditions, yield of the reaction products, and quantitative analysis results of Examples 1 to 11 and Comparative Examples 1 to 3 are shown in Tables 1 and 2.

Table 1

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| (A-1) Polyamide 66 | g | 20.03 | 20.01 | 20.00 | 20.02 | 19.96 | 12.00 | - | - | - | - | - |
| (A-2) Polyamide 66 waste | g | - | - | - | - | - | - | 20.03 | - | - | - | - |
| (A-3) Polyamide 66 waste | g | - | - | - | - | - | - | - | 21.94 | - | - | - |
| (A-4) Polyamide 66 waste | g | - | - | - | - | - | - | - | - | 28.61 | - | - |
| Polyamide 66 content | g | 20.03 | 20.01 | 20.00 | 20.02 | 19.96 | 12.00 | 20.03 | 20.01 | 20.03 | 20.04 | 20.02 |
| X | mmol | 88.50 | 88.41 | 88.37 | 88.46 | 88.19 | 53.02 | 88.50 | 88.41 | 88.49 | 88.55 | 88.46 |
| (B-1) Aqueous sodium hydroxide solution | 9 | 68.24 | 67.37 | 67.60 | 67.82 | 67.55 | 64.86 | 67.64 | 67.96 | 67.45 | - | - |
| Sodium hydroxide content | g | 7.46 | 7.11 | 7.41 | 7.44 | 7.40 | 4.45 | 7.48 | 7.46 | 7.42 | - | - |
| (B-2) Aqueous disodium carbonate solution | 9 | - | - | - | - | - | - | - | - | - | 68.61 | - |
| Disodium carbonate content | g | - | - | - | - | - | - | - | - | - | 9.85 | - |
| (B-3) Aqueous sodium hydrogen carbonate solution | 9 | - | - | - | - | - | - | - | - | - | - | 75.50 |
| Sodium hydrogen carbonate content | 9 | - | - | - | - | - | - | - | - | - | - | 15.61 |
| $Y_1$ | mmol | 186.51 | 177.76 | 185.26 | 186.01 | 185.01 | 111.26 | 187.01 | 186.51 | 185.51 | 185.87 | 185.82 |
| Deionized water | g | 60.78 | 60.26 | 60.19 | 60.38 | 60.15 | 60.41 | 60.16 | 60.50 | 60.03 | 58.76 | 59.89 |

EP 4 610 295 A1

(continued)

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| $Y_1/X$ | - | 2.11 | 2.01 | 2.10 | 2.10 | 2.10 | 2.10 | 2.11 | 2.11 | 2.10 | 2.10 | 2.10 |
| Z | - | 3.41 | 3.37 | 3.38 | 3.39 | 3.38 | 5.41 | 3.38 | 3.40 | 3.37 | 3.42 | 3.77 |
| Reaction temperature | °C | 280 | 280 | 230 | 300 | 280 | 280 | 280 | 280 | 280 | 280 | 280 |
| Reaction time | min | 40 | 40 | 15 | 40 | 20 | 40 | 40 | 40 | 40 | 40 | 40 |
| Diamine yield (X') (GC) | mol% | 98 | 93 | 93 | 97 | 94 | 98 | 95 | 98 | 93 | 70 | 54 |
| Dicarboxylic acid yield (IC) | mol% | 95 | 93 | 94 | 95 | 94 | 99 | 97 | 88 | 91 | 72 | 69 |
| Amino alcohol yield (Y') (GC) | mol% | 0.14 | 0.15 | 0.04 | 0.18 | 0.06 | 0.18 | 0.04 | 0.31 | 0.35 | 0.00 | 0.10 |
| $Y'/X'$ | $\times 10^{-3}$ | 1.4 | 1.6 | 0.4 | 1.8 | 0.6 | 1.8 | 0.4 | 3.2 | 3.8 | 0.0 | 1.9 |

Table 2

| | | Comparative Example 1 | Comparative Example 2 | Comparative Example 3 |
|---|---|---|---|---|
| (A-1) Polyamide 66 (X) | g | 20.07 | 20.04 | 20.03 |
| | mmol | 88.68 | 88.55 | 88.50 |
| (B-1) Aqueous sodium hydroxide solution | 9 | 69.16 | 68.16 | 67.92 |
| Sodium hydroxide | g | 8.88 | 7.43 | 7.42 |
| $Y_1$ | mmol | 222.02 | 185.76 | 185.51 |
| Deionized water | g | 60.28 | 60.73 | 60.50 |
| $Y_1/X$ | - | 2.50 | 2.10 | 2.10 |
| Z | | 3.45 | 3.40 | 3.39 |
| Reaction temperature | °C | 280 | 280 | 220 |
| Reaction time | min | 40 | 60 | 60 |
| Diamine yield (X') (GC) | mol% | 75 | 93 | 93 |
| Dicarboxylic acid yield (IC) | mol% | 99 | 94 | 97 |
| Amino alcohol yield (GC) | mol% | 0.58 | 0.62 | 0.52 |
| Y'/X' | $\times 10^{-3}$ | 7.7 | 6.7 | 5.6 |

From Examples 1 to 11, it is understood that when a polyamide is brought into contact with an aqueous solution containing a preferable amount of an alkaline metal hydroxide or alkaline metal carbonate, it is possible to make the hydrolysis sufficiently proceed, increase the yields of the diamine and the dicarboxylic acid, and reduce the amount of the amino alcohol represented by Chemical formula (1).

In Comparative Example 1 in which $Y_1/X$ was larger than the preferable range, the yield of the diamine decreased and the production amount of the amino alcohol increased as compared with Example 1 in which the reaction was performed under substantially the same conditions except for $Y_1/X$. When the amount of the alkaline metal hydroxide was excessive, it was difficult to obtain both the diamine and the dicarboxylic acid in good yield even though the conditions of the reaction temperature and the reaction time were satisfied, and the production amount of the amino alcohol increased.

Meanwhile, in Comparative Example 2 in which the reaction was performed for a long time exceeding the preferable range, the production amount of the amino alcohol increased as compared with Example 1 in which the reaction was performed under substantially the same conditions except for the reaction time.

In Comparative Example 3 in which the reaction temperature was lower than 230°C, the diamine and the dicarboxylic acid were obtained in good yield under the reaction time of 60 minutes, but the production amount of the amino alcohol remained large.

As described above, it is understood that when a polyamide composition is brought into contact with an aqueous solution containing a preferable amount of an alkaline metal hydroxide and/or alkaline metal carbonate at a temperature of 230°C or higher for 0.1 to 45 minutes, it is possible to make the hydrolysis sufficiently proceed, increase the yields of the diamine and the dicarboxylic acid, and reduce the amount of the amino alcohol represented by Chemical formula (1). In addition, in the case where the reaction temperature is lower than the preferable range, as described in Patent Literature 1 and Non Patent Literature 1, the reaction takes a long time, and the amount of the amino alcohol is larger than that in the case where the reaction is performed at a preferable reaction temperature.

As for a case where polyamide 66 is selected as the polyamide, 6-amino-1-hexanol that is an amino alcohol derived from hexamethylenediamine has a boiling point of 225°C, which is close to 204°C, that is, the boiling point of hexamethylenediamine. Therefore, when the reaction mixture contains a large amount of the amino alcohol, the separation and removal by distillation becomes more difficult, and it is considered that recycling of the polyamide becomes difficult.

[Reference Example 1]

In 12.6 g of deionized water, 5.15 g of hexamethylenediamine and 6.47 g of adipic acid were dissolved to prepare a salt solution. The salt solution was charged into a reaction vessel, and the reaction vessel was sealed and purged with nitrogen. A heater on the outer periphery of the reaction vessel was set to a temperature of 290°C, and heating was started.

After the internal pressure reached 1.75 MPa, the internal pressure was maintained at 1.75 MPa while water was released to the outside of the system, and the temperature was raised until the internal temperature reached 237°C. After the internal temperature reached 237°C, the internal pressure was adjusted to normal pressure over 1 hour (internal temperature when the pressure reached normal pressure: 257°C). Subsequently, the reaction vessel was held while nitrogen was fed into the vessel (nitrogen flow) for 60 minutes, thereby obtaining polyamide 66 (maximum attained temperature: 274°C). The obtained polyamide 66 had a number average molecular weight of 14300 g/mol and a melting point of 262°C.

[Example 12]

A diamine was extracted with isobutanol from a reaction mixture obtained in the same manner as in Example 1, concentrated using an evaporator, and then distilled at 84 to 90°C and $3 \pm 1$ hPa to give a crude diamine composition. The crude diamine composition was distilled again at 84 to 90°C and $3 \pm 1$ hPa to give a diamine composition. The diamine composition had an amino alcohol content of $0.80 \times 10^{-5}$ mol per 1 g of a diamine.

To an aqueous dicarboxylic acid solution from which the diamine had been removed by extraction, 15 mL of a 35% aqueous hydrochloric acid solution was added to give a slurry solution in which the dicarboxylic acid was precipitated. The slurry solution was heated in an oil bath at 80°C to form a uniform solution, and then the solution was allowed to stand at room temperature for 12 hours to precipitate crude adipic acid. To the crude adipic acid recovered by vacuum filtration, deionized water was added in an amount of two times the mass of the crude adipic acid, and the resulting mixture was heated again in an oil bath at 80°C to form a uniform solution. Thereafter, adipic acid precipitated by allowing the solution to stand at room temperature for 12 hours was recovered by vacuum filtration, and dried in a vacuum oven set at 110°C for 12 hours to give adipic acid.

Polyamide 66 was produced in the same manner as in Reference Example 1 except that 5.14 g of the diamine composition and 6.50 g of adipic acid thus obtained were used as polymerization raw materials. The obtained regenerated polyamide 66 had a number average molecular weight of 15300 g/mol, a melting point of 262°C, and an amount of amino alcohol terminal groups of $0.41 \times 10^{-5}$ mol/g.

[Example 13]

As for a reaction mixture obtained in the same manner as in Example 3, distillation was performed twice in the same manner as in Example 12 to give a diamine composition. The diamine composition had an amino alcohol content of $0.010 \times 10^{-5}$ mol per 1 g of a diamine. In addition, adipic acid and polyamide 66 were produced in the same manner as in Example 12. The obtained regenerated polyamide 66 had a number average molecular weight of 15000 g/mol, a melting point of 262°C, and an amount of amino alcohol terminal groups of 0 mol/g (below the detection limit of gas chromatography).

[Comparative Example 4]

A diamine composition and adipic acid were produced in the same manner as in Example 12 from a reaction mixture obtained in the same manner as in Comparative Example 1. The diamine composition had an amino alcohol content of $6.4 \times 10^{-5}$ mol per 1 g of a diamine. Polyamide 66 was produced in the same manner as in Reference Example 1 except that the diamine composition and adipic acid were used as polymerization raw materials. The obtained regenerated polyamide 66 had a number average molecular weight of 11000 g/mol, a melting point of 261°C, and an amount of amino alcohol terminal groups of $3.3 \times 10^{-5}$ mol/g.

Therefore, from Reference Example 1, Example 12, and Example 13, it was found that the polyamide obtained by repolymerizing adipic acid and the diamine composition of the present invention, which were obtained by depolymerizing a polyamide, has a number average molecular weight that is larger than that of a polyamide obtained by polymerizing a reagent diamine and a reagent dicarboxylic acid.

Meanwhile, from Reference Example 1 and Comparative Example 4, it was found that the polyamide obtained by repolymerizing the diamine composition having an amino alcohol content larger than $6.0 \times 10^{-5}$ mol per 1 g of a diamine had $3.0 \times 10^{-5}$ mol/g or more of a structure derived from the amino alcohol at the terminal, and had a small number average molecular weight. It is presumed that the polymerization is inhibited by the amino alcohol in the diamine composition. 6-Amino-1-hexanol that is an amino alcohol derived from a hexamethylenediamine residue has a boiling point of 225°C, which is close to 204°C, that is, the boiling point of hexamethylenediamine. Therefore, it is considered that the reaction mixture contained a large amount of the amino alcohol, and the removal by distillation was difficult.

17

Industrial Applicability

The diamine composition of the present invention can be suitably used as a repolymerization raw material for a polyamide since it contains a small amount of impurities. In addition, in particular, obtaining the diamine composition of the present invention by depolymerization of a polyamide can contribute to recycling of resources and reduction of greenhouse gas emission.

**Claims**

1. A diamine or a diamine composition obtained by depolymerizing a polyamide, comprising $6.0 \times 10^{-5}$ mol or less of an amino alcohol represented by Chemical formula (1) per 1 g of a diamine:

   Chemical formula (1):  $H_2N\text{-}R\text{-}OH$

   wherein R is a residue containing at least one group selected from an aliphatic group, an alicyclic group, and an aromatic group each having 3 to 12 carbon atoms.

2. The diamine composition according to claim 1, wherein an amount of the amino alcohol is $0.010 \times 10^{-5}$ mol or more per 1 g of the diamine.

3. A polyamide comprising, as a polymerization raw material, a diamine or a diamine composition obtained by depolymerizing a polyamide, and comprising $3.0 \times 10^{-5}$ mol/g or less of a terminal structure represented by Chemical formula (2):

   Chemical formula (2):  $-NH\text{-}R\text{-}OH$

   wherein R is a residue containing at least one group selected from an aliphatic group, an alicyclic group, and an aromatic group each having 3 to 12 carbon atoms.

4. A molded article, a fiber, a film, or a sheet comprising the polyamide according to claim 3.

5. A method for producing a diamine and/or a dicarboxylic acid, the method comprising:
   mixing a polyamide composition (A) containing a polyamide with an aqueous solution (B) of an alkaline (earth) metal compound selected from a hydroxide, an oxide, a carbonate, and a mixture containing two or more thereof to satisfy Formula (1), the polyamide composition (A) containing X mol of a dicarboxylic acid residue, the aqueous solution (B) containing $Y_1$ mol of an alkaline metal ion and/or $Y_2$ mol of an alkaline earth metal ion, and holding a resulting mixture at a temperature of 230°C or higher for 0.1 to 45 minutes:

   $$\text{Formula (1): } 1.6 \leq (Y_1 + 2 \times Y_2)/X < 2.4.$$

6. The production method according to claim 5, wherein a temperature in the holding is equal to or higher than a melting point of the polyamide composition (A) that is determined by differential scanning calorimetry and lower than 400°C.

7. The production method according to claim 5, wherein in a mass ratio of the polyamide composition (A) to the aqueous solution (B) of the alkaline (earth) metal compound selected from the hydroxide, the oxide, the carbonate, and the mixture containing two or more thereof, that is, 1 : Z, Z is 1.0 or more and 10.0 or less.

8. The production method according to claim 5, wherein each of the diamine and the dicarboxylic acid is produced from the polyamide composition (A) in a yield of 70 mol% or more.

9. The production method according to claim 5, wherein when amounts of a diamine and an amino alcohol represented by Chemical formula (1) that are contained in a reaction mixture (C) obtained by contacting the polyamide composition (A) with the aqueous solution (B) of the alkaline (earth) metal compound selected from the hydroxide, the oxide, the carbonate, and the mixture containing two or more thereof are represented by X' mol and Y' mol, respectively, Y'/X' is $4.0 \times 10^{-3}$ or less:

Chemical formula (1): H$_2$N-R-OH

wherein R is a residue containing at least one group selected from an aliphatic group, an alicyclic group, and an aromatic group each having 3 to 12 carbon atoms.

10. The production method according to claim 5, wherein the polyamide composition (A) is a waste.

11. A method for producing the diamine or the diamine composition according to claim 1, the method comprising the following steps (1) to (3) in this order:

(1) bringing a polyamide composition (A) into contact with an aqueous solution (B) of an alkaline (earth) metal compound selected from a hydroxide, an oxide, a carbonate, and a mixture containing two or more thereof to produce a diamine and a dicarboxylate;
(2) separating the diamine and the dicarboxylate produced in step (1) from each other; and
(3) distilling the diamine separated in step (2).

12. A method for producing a polyamide, the method comprising polycondensing a raw material containing the diamine or the diamine composition obtained by the method according to claim 11.

## INTERNATIONAL SEARCH REPORT

| International application No. |
|---|
| **PCT/JP2023/038787** |

### A. CLASSIFICATION OF SUBJECT MATTER

*C08G 69/46*(2006.01)i; *C07C 51/06*(2006.01)i; *C07C 55/14*(2006.01)i; *C07C 209/00*(2006.01)i; *C07C 211/12*(2006.01)i; *C08G 69/28*(2006.01)i; *C08G 69/44*(2006.01)i; *C08G 69/48*(2006.01)i; *C08J 11/16*(2006.01)i

FI: C08G69/46; C08G69/44; C08G69/28; C07C209/00; C07C55/14; C07C51/06; C08J11/16; C08G69/48; C07C211/12

According to International Patent Classification (IPC) or to both national classification and IPC

### B. FIELDS SEARCHED

Minimum documentation searched (classification system followed by classification symbols)

C08G69/46; C07C51/06; C07C55/14; C07C209/00; C07C211/12; C08G69/28; C08G69/44; C08G69/48; C08J11/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | CN 1611477 A (MINTE NYLON INDUSTRY CO., LTD., NINGBO) 04 May 2005 (2005-05-04)<br>　　entire text | 1-12 |
| A | CN 107056625 A (CHAOHU DINGSHENG FISHING GEAR CO., LTD.) 18 August 2017 (2017-08-18)<br>　　entire text | 1-12 |
| A | JP 10-510295 A (E.I. DU PONT DE NEMOURS AND COMPANY) 06 October 1998 (1998-10-06)<br>　　entire text | 1-12 |
| A | JP 39-23390 B1 (IMPERIAL CHEMICAL INDUSTRIES LIMITED) 20 October 1964 (1964-10-20)<br>　　entire text | 1-12 |
| A | JP 2001-131282 A (KURARAY CO LTD) 15 May 2001 (2001-05-15)<br>　　entire text | 1-12 |

☐ Further documents are listed in the continuation of Box C.　　☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **15 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/038787**

| Patent document cited in search report | | | Publication date (day/month/year) | Patent family member(s) | Publication date (day/month/year) |
|---|---|---|---|---|---|
| CN | 1611477 | A | 04 May 2005 | (Family: none) | |
| CN | 107056625 | A | 18 August 2017 | (Family: none) | |
| JP | 10-510295 | A | 06 October 1998 | US 5750791 A entire text WO 1997/000846 A1 | |
| JP | 39-23390 | B1 | 20 October 1964 | (Family: none) | |
| JP | 2001-131282 | A | 15 May 2001 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07507556 A
- JP 2001302597 A
- WO 2023149514 A

**Non-patent literature cited in the description**

- *Polymer Degradation and Stability*, 2004, vol. 83, 389-393